# EUROPÄISCHE PATENTSCHRIFT

(11) **EP 2 226 381 B1**
(45) Veröffentlichungstag und Bekanntmachung des Hinweises auf die Patenterteilung: **09.03.2016**
(21) Anmeldenummer: 10152393.4
(22) Anmeldetag: 07.11.2003
(51) Int. Cl.: C12P 21/08, C12N 5/07

(54) **VERFAHREN ZUR KULTIVIERUNG VON ZELLEN ZUR PRODUKTION VON SUBSTANZEN**
METHOD FOR CULTIVATING CELLS FOR THE PRODUCTION OF COMPOUNDS
PROCEDE POUR CULTIVER DES CELLULES POUR LA PRODUCTION DE SUBSTANCES

(30) Priorität: 27.11.2002 DE 10255508
(43) Veröffentlichungstag der Anmeldung: 08.09.2010
(62) Teilanmeldung aus: 03767406.6
(73) Patentinhaber: F. Hoffmann-La Roche AG, 4070 Basel (CH)
(72) Erfinder: Essers, Ruth, 51063 Koeln (DE); Gaetgens, Jochen, 52438 Juelich (DE); Link, Thomas, 82377 Penzberg (DE); Noll, Thomas, 33615 Bielefeld (DE); Skopnik, Kerstin, 51465 Bergisch Gladbach (DE); Wandrey, Christian, 52428 Juelich (DE)
(74) Vertreter: Burger, Alexander

(56) Entgegenhaltungen:
- WO-A1-98/41611

## Beschreibung

Die Erfindung betrifft ein Verfahren zur Kultivierung von Zellen zur Produktion von Immunglobulinen oder Fragmenten von Immunglobulinen nach dem Oberbegriff des Anspruchs 1.

Bei der Produktion von Substanzen, insbesondere Proteinen, werden Zellkulturen in fermentativen Prozessen eingesetzt. Es können dabei Prozesse unterschieden werden, bei denen die Zellkulturen genetisch unverändert sind und eigene Stoffwechselprodukte bilden und bei denen die Organismen genetisch so modifiziert sind, daß sie entweder eigene Substanzen, beispielsweise Proteine, vermehrt, oder fremde Substanzen beispielsweise Proteine produzieren. Die die Substanzen produzierenden Organismen werden dabei mit einem Nährmedium versorgt, welches das Überleben der Organismen garantiert und die Produktion der gewünschten Zielverbindung ermöglicht. Für diese Zwecke sind eine Vielzahl von Kulturmedien bekannt, die eine Fermentation ermöglichen. Einer der wichtigsten Bestandteile der Kulturmedien ist die Glukose. Nach dem Stand der Technik ist man regelmäßig bemüht in einem Fermentationsansatz eine Mindestkonzentration an Glukose aufrecht zu erhalten, um die Ausbeute an Zielverbindung zu optimieren. Die japanische Patentanmeldung 001 101 882 A offenbart ein Kultivierungsverfahren für Säugetierzellen bei dem eine Mindestkonzentration von 0,2 mmol/l an Glukose aufrechterhalten wird. Die US 544 39 68 offenbart ein Kultivierungsverfahren, bei dem eine Glukoselimitierung erfolgt. Das Verfahren führt jedoch nicht zu einer höheren spezifischen Produktionsrate der Zellen gegenüber der nicht limitierenden Fütterung.

Die WO 98/41611 offenbart ein dreistufiges Verfahren zur Kultivierung von Säugetierzellen unter Glukoselimitierung, welches eine Batch-Phase zur Reduktion der Glukose-Konzentration beinhaltet, gefolgt von einer Fed-Batch-Phase, in der es durch die Glukoselimitierung zu einer Umstellung des Zellmetabolismus kommt ("metabolic shift") und schließlich eine Phase der kontinuierlichen Prozessführung. Der Grad der Glukoselimitierung wird in dem Dokument definiert durch das Verhältnis von gebildetem Laktat zu verbrauchter Glukose (< 1).

Es ist die Aufgabe der Erfindung ein Verfahren zur Kultivierung von Zellen zu schaffen, mit dem die Produktivität einer einzelnen Zelle an Produkt gesteigert wird und bei dem hohe Zelldichten ermöglicht werden. Es soll eine hohe Raum-/Zeit-Ausbeute an Produkt ermöglicht werden.

Das Verfahren soll besonders einfach in der Durchführung, mit minimalem Meß- und Regelaufwand verbunden, und besonders wirtschaftlich sein.

Ausgehend vom Oberbegriff des Anspruchs 1 wird die Aufgabe überraschenderweise dadurch gelöst, daß die Kultivierung einer Immunglobulin oder eines Fragmentes eines Immunglobulins produzierende Zellinie unter Zufuhr eines Nährmediums in der Weise erfolgt, daß sich in der Kulturlösung eine Glukoselimitierung einstellt. Der Grad der Glukoselimitierung kann definiert werden, als das Verhältnis der beobachteten spezifischen Glukoseverbrauchsrate zur maximal für diese Zellen bekannten spezifischen Glukoseverbrauchsrate. Der Grad der Glukoselimitierung DGL = qGlc/qGlcₘₐₓ (qGlc = momentane beobachtete, spezifische Glukoseverbrauchsrate; qGlcₘₐₓ = maximal für diese Zellen bekannten spezifischen Glukoseverbrauchsrate). DGL liegt in den Grenzen zwischen DGL_{Erhaltung} und 1, wobei DGL_{Erhaltung} völlige Wachstumslimitierung bedeutet und 1 bedeutet keinerlei Limitierung bzw. völliger Glukoseüberschuß.

Zusammen mit der Glukoselimitierung erfolgt ein kontinuierlicher Rückgang der Restglukosekonzentration auf eine stationäre Konzentration in der Kulturlösung, die größer 0 mmol/l, jedoch kleiner als 1 mmol/l, bevorzugt kleiner als 0,5 mmol/l ist. Es ist zu beobachten, daß mit Sinken des DGL ein weiterer Anstieg der Lebendzelldichte im Kulturgefäß erfolgen kann. Mit zunehmender Glukoselimitierung konvergiert die Zelldichte dann gegen einen Maximalwert. Daraus resultiert, daß der Grad der Glukoselimitierung gegen einen minimalen Wert konvergiert, dabei ist der DGL erfindungsgemäß größer oder gleich dem DGL, welcher zur Erhaltung der Zelle führt, (maintenance Stoffwechsel) DGL_{Erhaltung} = qGlC_{Erhaltung}/qGlCₘₐₓ (qGlc_{Erhaltung} = bei reinem Erhaltungsstoffwechsel beobachtete spezifische Glukoseverbrauchsrate; qGlcₘₐₓ = maximal für diese Zellen bekannte spezifische Glukoseverbrauchsrate), und kleiner 0,5, vorzugsweise kleiner 0,4, besonders bevorzugt kleiner 0,3.

Charakteristisch ist jedoch, daß mit der Abnahme der Glukosekonzentration keine Abnahme der Zellkonzentration in der Lösung erfolgt. Mit zunehmender Glukoselimitierung, also bei sinkendem DGL-Wert, erhöht sich die spezifische Produktivität einer Zelle. Da die Lebendzelldichte im Kulturgefäß nicht absinkt, führt dies zu einer Erhöhung der Raum-Zeit Ausbeute. Mit Eintreten der Glukoselimitierung geht phänomenologisch eine Erniedrigung der spezifischen Laktatbildungsrate einher. Die Laktatbildungsrate konvergiert gegen einen Minimalwert. Dies führt dazu, dass die Restlaktatkonzentration im Kulturgefäß absinkt, maximal gegen 0 geht. Mit der Glukoselimitierung kommt es also zu einer Umstellung des Zellmetabolismus.

Wichtig ist dabei, daß es vor Einsetzen der Glukoselimitierung zu keiner weiteren Limitierung durch andere Substrate kommt. Daher muß das Wachstumsmedium so beschaffen sein, daß Glukose zuerst limitiert ist.

Mit dem erfindungsgemäßen Verfahren wird die Raum-/ZeitAusbeute bei gegebener Zelldichte erhöht. Durch das erfindungsgemäße Verfahren wird die pro Zelle zur Verfügung stehende Menge an Glukose derart vermindert, daß die Glukose überwiegend in den Erhaltungsstoffwechsel und verbunden damit das Produkt und weniger in Zellwachstum eingeht. Dadurch, daß ein geringer Medienzufluss nötig ist, werden Kosten für Glukose gespart, da weniger Glukose benötigt wird. Zudem wird eine sehr hohe Produktkonzentration erreicht. Dies kann zur Senkung der Aufarbeitungskosten führen. Das erfindungsgemäße Verfahren ermöglicht insbesondere die Steigerung der Produktion von Immunglobulinen oder Fragmenten von Immunglobulinen, ohne daß eine Zellinie für die Umsetzung des erfindungsgemäßen Verfahrens zusätzlich genetisch verändert werden muss. Die Erhöhung des Produkttiters ermöglicht die Produktion einer gewünschten Menge an Immunglobulinen oder Fragmenten von Immunglobulinen in einem kleineren Kultivierungsvolumen, was zu geringeren Investitionskosten führt.

Das erfindungsgemäße Verfahren wird entweder als Batch gestartet und als kontinuierlicher Prozess fortgeführt, oder es besteht aus einer Batch und einer Fed-Batch Verfahrensweise, wobei das Verfahren als Batch gestartet und als Fed-Batch Prozess fortgeführt wird.

Die Zellen sollen vorzugsweise in kontinuierlicher Verfahrensweise mit Zellrückhaltung, z. B. Spinfilter (Perfusionskultur) kultiviert werden. Dabei sind alle gängigen Arten von Kulturgefäßen, wie zum Beispiel Rührkessel, und Zellrückhaltemechanismen, wie beispielsweise Spinfilter, Ultraschall oder Settler geeignet. Vorzugsweise sollte das Kultursystem hohe Zelldichten ermöglichen. Vorzugsweise wird eine Zellrückhaltung realisiert, damit die Zelldichte bei Auftreten der Glukoselimitierung nicht absinken kann. Dies führt dazu, dass bei steigender Lebendzelldichte und gleichbleibender Glukosezufütterung, der DGL weiter verringert wird. Die hohe Zelldichte ermöglicht ein Absinken des DGL unter einen Wert von 0,4 bei einer eingestellten Durchflussrate in einer Größenordnung der maximalen Wachstumsrate. So können beispielhaft Durchflussraten von 0,03 - 0,05 h⁻¹ für die verwendete CHO MUC2-GFP-C-term Zelle, sowie die verwendete CHO/MUC1-IgG2a PH3744/25 Zelle angewendet werden.

Um eine Verringerung des DGL zu erreichen, kann die Fütterungsstrategie mit Glukose demnach wie folgt erfolgen: Die Menge an zugefütterter Glukose wird nicht mit zunehmender Lebendzelldichte erhöht, um eine Glukoselimitierung zu vermeiden. Dabei sollte die Menge an zugefütterter Glukose so gewählt sein, dass der DGL die erforderlichen Werte unterschreitet, nämlich DGL kleiner ≤ 0,5, vorzugsweise ≤ 0,4, besonders bevorzugt ≤ 0,3. Daraus resultiert, daß die Menge zugefütterter Glukose vorzugsweise nicht grösser als 50 %, besonders bevorzugt nicht grösser als 35 % dessen ist, was die im System bei herkömmlicher, nicht glukoselimitierter Prozeßführung zu erwartende Lebendzellzahl maximal verbrauchen kann. Nach Umstellung des Zellmetabolismus (Laktatstoffwechel und Produktivität) kann die Menge zugefütterter Glukose langsam erhöht werden, sollte dabei jedoch nicht einen DGL von größer als 0,5, vorzugsweise größer als 0,4 ermöglichen. Dies führt zu einer weiteren Erhöhung der Lebendzelldichte bei gleichbleibend hoher Produktivität und damit erhöhter Raum-/Zeit-Ausbeute. Die Menge zugeführter Glukose kann in einem kontinuierlichen Prozeß durch die Medienzuflußrate und die Glukosekonzentration im Zufütterungsmedium beeinflußt werden. Maßgeblich ist, dass der Massenfluss an zugeführter Glukose während des Prozesses nicht oder nur in solchem Maße erhöht wird, daß der DGL einen Wert von kleiner 0,5, vorzugsweise kleiner 0,4 erreicht oder unterschreitet und dieser dann nicht mehr überschritten wird.

Vorteilhafte Weiterbildungen der Erfindung sind in den Unteransprüchen angegeben.

Im Folgenden soll die Erfindung in ihren Einzelheiten dargestellt werden.

Die Figuren zeigen beispielhafte Versuchsergebnisse.

Es zeigt:
- Fig.1:: Zunahme der vitalen Zellzahl [ml⁻¹] und Darstellung der Mediendurchflußrate [h⁻¹] gegen die Prozesszeit [h] für die Produktion von MUC1-IgG2a aus CHO MUCl/IgG2a PH3744/25 Zellen im Perfusionsreaktor.
- Fig.2:: Spezifische Produktivität an MUC1-IgG2a [µg/(h*E9 Zellen)] und DGL gegen die Prozesszeit im Perfusionsreaktor.
- Fig.3:: Zunahme der vitalen Zellzahl [ml⁻¹] und mM Restglukose, aufgetragen gegen die Prozesszeit [h] für die Produktion von MUC1-IgG2a aus CHO MUC1/IgG2a PH3744/25 Zellen im Perfusionsreaktor.
- Fig.4:: Glukose- und Laktatkonzentration sowie Konzentration von Glukose im Medienzulauf [mmol/l], aufgetragen gegen die Prozesszeit [h] für die Produktion von MUC1-IgG2a aus CHO MUC1/IgG2a PH3744/25 Zellen im Perfusionsreaktor.
- Fig.5:: Zunahme der Konzentration an MUC1-IgG2a [µg/ml] und qMUC1-IgG2a [µg/(h*E9 Zellen)] gegen die Zeit [h] für die Produktion von MUC1-IgG2a aus CHO MUC1/IgG2a PH3744/25 Zellen im Perfusionsreaktor.
- Fig.6:: Zunahme der vitalen Zellzahl [ml⁻¹] und Darstellung der Mediendurchflußrate [h⁻¹] gegen die Prozesszeit [h] für die Produktion von MUC2-GFP-C-term aus CHO MUC2-GFP-C-term Zellen im Perfusionsreaktor.
- Fig.7:: Spezifische Produktivität an MUC2-GFP-C-term [nmol/(h*E9 Zellen)] und DGL gegen die Prozesszeit im Perfusionsreaktor.
- Fig.8:: Zunahme der vitalen Zellzahl [ml⁻¹] und Restglukose [mM], aufgetragen gegen die Prozesszeit [h] für die Produktion von MUC2-GFP-C-term aus CHO MUC2-GFP-C-term Zellen im Perfusionsreaktor.
- Fig.9:: Glukose- und Laktatkonzentration sowie Konzentration von Glukose im Medienzulauf [mmol/l], aufgetragen gegen die Prozesszeit [h] für die Produktion von MUC2-GFP-C-term aus CHO MUC2-GFP-C-term Zellen im Perfusionsreaktor.
- Fig.10:: Zunahme der Konzentration an MUC2-GFP-C-term [nM] und qMUC2-GFP-C-term [nmol/(h*E9 Zellen)] gegen die Zeit [h] für die Produktion von MUC2-GFP-C-term aus CHO MUC2-GFP-C-term Zellen im Perfusionsreaktor.

Weiterhin zeigt Tabelle 1 die Versuchsdaten aus der Anwendung des erfindungsgemäßen Verfahrens mit der CHO MUC1/IgG2a PH 3744/25 Zelle.

In Tabelle 2 sind die Versuchsdaten aus der Anwendung des erfindungsgemäßen Verfahrens mit der CHO MUC2-GFP-C-term Zelle dargestellt.

Die erfindungsgemäße Verfahrensweise kann mit verschiedenen Produktionszellinien durchgeführt werden. Die Zellinien können als Wildtyp oder als genetisch modifizierte, rekombinante Zellen eingesetzt werden. Die genetische Modifikation kann beispielsweise durch Insertion von zusätzlichen Genen des gleichen Organismus oder eines anderen Organismus in die DNA, oder einen Vektor erfolgen, oder in der Verstärkung der Aktivität bzw. Expression eines Gens durch Einbringen eines wirksameren Promotors, zum Beispiel aus CMV. Die Gene können für verschiedene Proteine kodieren, beispielsweise für Proteine, wie Fusionsproteine oder für Antikörper.

Folgende Zellinien können beispielhaft genannt werden: Säugerzellen, wie CHO Zellinien, wie zum Beispiel CHO-K1, BHK, wie BHK-21, Hybridoma, NS/0, andere Myelomazellen und Insektenzellen oder andere höhere Zellen. Besonders bevorzugt ist die Verwendung von Zellen, die nicht vorzugsweise wachstumsgekoppelt produzieren.

Eine rekombinante CHO Zellinie deren Produktivität mit der erfindungsgemäßen Verfahrensweise gesteigert werden kann ist die Zellinie CHO MUC1/IgG2a, PH 3744/25, mit der das Glykoprotein MUC1-IgG2a sekretiert werden kann. Eine weitere CHO Zellinie, nämlich CHO MUC2-GFP-C-term, ist befähigt ein Fusionsprotein MUC2-GFP-C-term gesteigert zu sekretieren, wenn sie der erfindungsgemäßen Verfahrensweise unterzogen wird.

Als Kulturmedium kann prinzipiell jedes glukosehaltige Medium eingesetzt werden, welches bezüglich anderer Komponenten nicht limitierend ist. Beispielhaft kann ProCHO4-CDM genannt werden. Es können auch Medien basierend auf bekannten Rezepturen, wie zum Beispiel IMDM, DMEM oder Ham's F12 eingesetzt werden, die so auf die erfindungsgemäße Verfahrensweise optimiert wurden, daß lediglich Glukoselimitierung auftritt. Dies kann zum Beispiel dadurch erreicht werden, dass andere Komponenten im Verhältnis zur Glukose höher konzentriert werden. Generell ist es auch möglich die Glukose separat vom Medium zu zu dosieren.

Der pH Bereich liegt vorzugsweise zwischen 6,7 - 7,7, besonders bevorzugt zwischen 7 - 7,3. Jedoch sind auch andere pH-Bereiche denkbar.

Der Temperaturbereich liegt vorzugsweise zwischen 35 °C - 38,5 °C, besonders bevorzugt bei 37 °C für CHO MUC1-IgG2a. Es sind aber auch andere Temperaturbereiche denkbar, wie beispielsweise < 35 °C, bei denen es nicht zur irreversiblen Zerstörung des Produkts kommt.

Mit dem erfindungsgemäßen Kultivierungsverfahren können Substanzen, wie Glykoproteine, Fusionsproteine, Antikörper, Proteine im Allgemeinen produziert werden, von denen beispielhaft MUC1-IgG2a, MUC2-GFP-C-term, EPO, Interferone, Cytokine, Wachstumsfaktoren, Hormone, PA, Immunglobuline oder Fragmente von Immunglobulinen, genannt werden können.

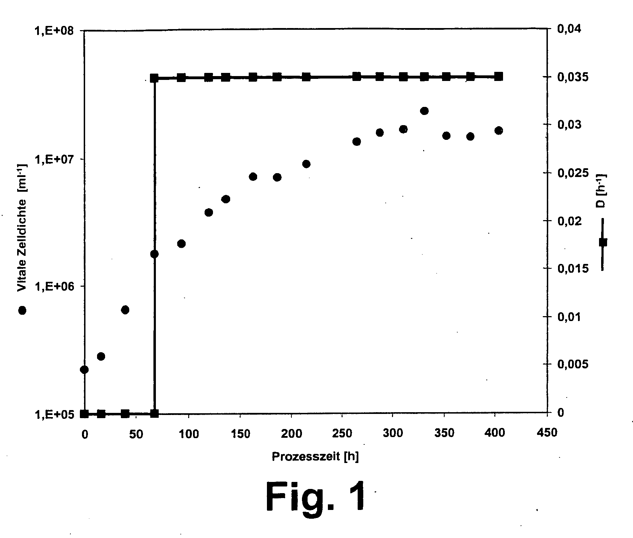

Figur 1 zeigt den Verlauf der Lebendzelldichte (cv) an CHO/MUC1-IgG2a Zellen und der Mediendurchflußrate (D) gegen die Prozesszeit (h) im Perfusionsreaktor. In ihr ist:
▪ Die Mediendurchflußrate (1/h) und
• die Lebendzelldichte (1/ml).

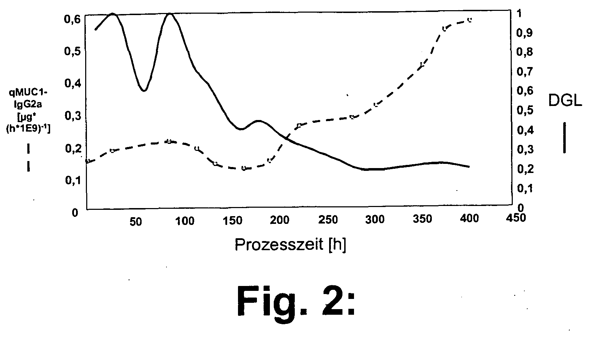

Figur 2 zeigt die spezifische Produktivität an MUC1-IgG2a (qMUC1-IgG2a) und DGL gegen die Prozesszeit im Perfusionsreaktor. In ihr ist:
Die spezifische Produktivität (µg/hE9 Zellen),
- DGL (degree of glucose limitation).

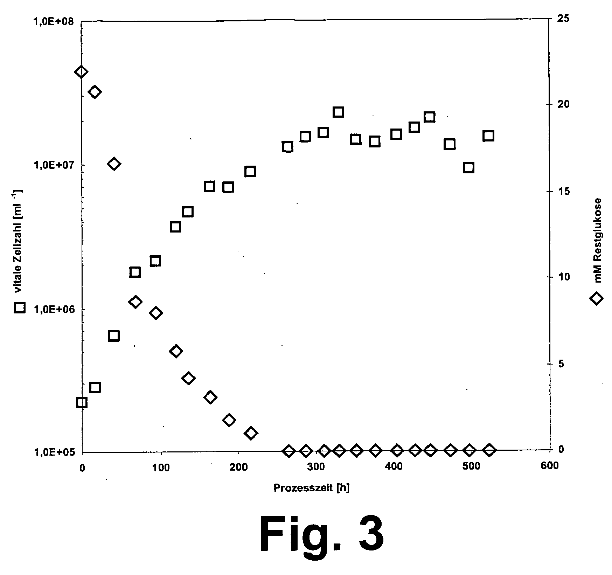

Figur 3 zeigt eine Graphik, in der auf der linken Seite die vitale Zellzahl [ml⁻¹] und auf der rechten Seite die Konzentration der Restglukose [mM] gegen die Prozesszeit [h] für die Produktion von MUC1-IgG2 in CHO MUC1/IgG2a PH3744/25 aufgetragen ist. In ihr ist:
□ vitale Zellzahl und
0 Glukose.

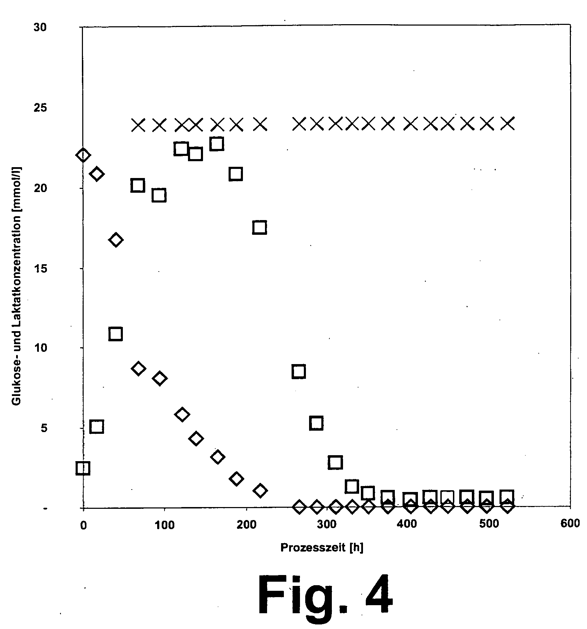

In Figur 4 ist die Glukose- und Laktatkonzentration sowie die Glukosekonzentration im Medienzulauf [mmol/l] gegen die Prozesszeit [h] aufgetragen. In ihr sind die Kurven mit
□ Laktatkonzentrationskurven und
0 Glukosekonzentrationskurven.
x 23,9 mmol/l Konzentration an Glukose im Medienzulauf (Durchflussrate von D = 0,035 h⁻¹).

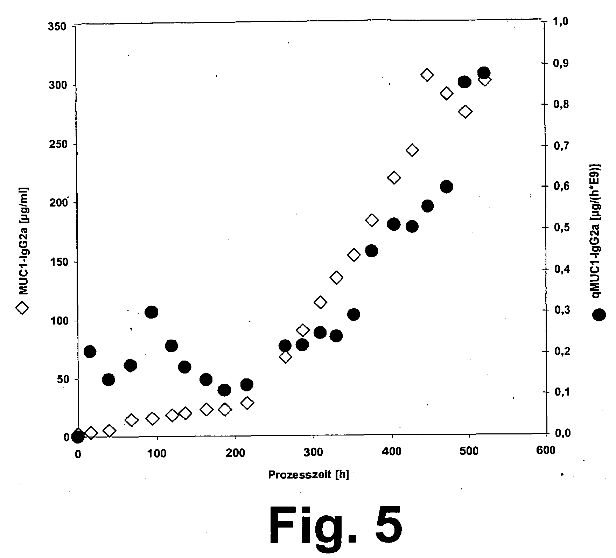

In Figur 5 ist die Konzentration von MUC1-IgG2a [µg/ml] auf der linken Seite sowie qMUC1-IgG2a [µg/(h*E9 Zellen)] auf der rechten Seite der Graphik gegen die Zeit [h] aufgetragen. In ihr sind:
• q spezifische Produktivität an MUC1-IgG2a (µg/hE9 Zellen) und
0 Konzentration an MUC1-IgG2a (mg/l).

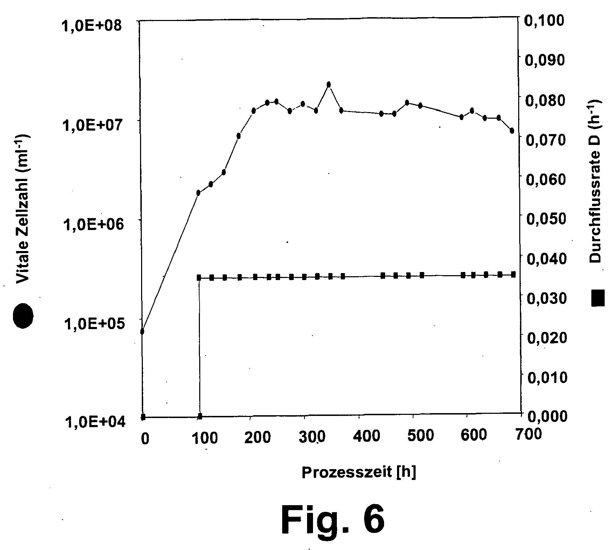

Figur 6 zeigt den Verlauf der Lebendzelldichte (cv) an CHO/MUC2-GFP Zellen und der Mediendurchflußrate (D) gegen die Prozesszeit (h) im Perfusionsreaktor. In ihr ist:
▪ Die Mediendurchflußrate (1/h) und
• die Lebendzelldichte (1/ml).

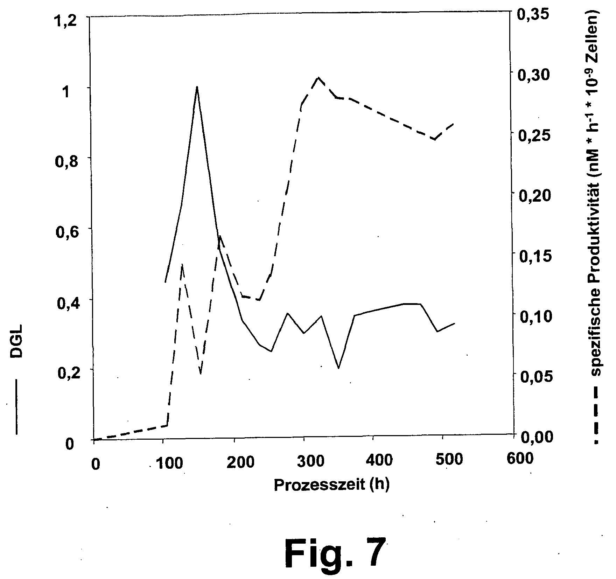

Figur 7 zeigt die spezifische Produktivität an MUC2-GFP-C-term (qMUC2-GFP-C-term) und DGL gegen die Prozeßzeit im Perfusionsreaktor. In ihr ist:
Die spezifische Produktivität (nmol/hE9 Zellen),
- DGL (degree of glucose limitation).

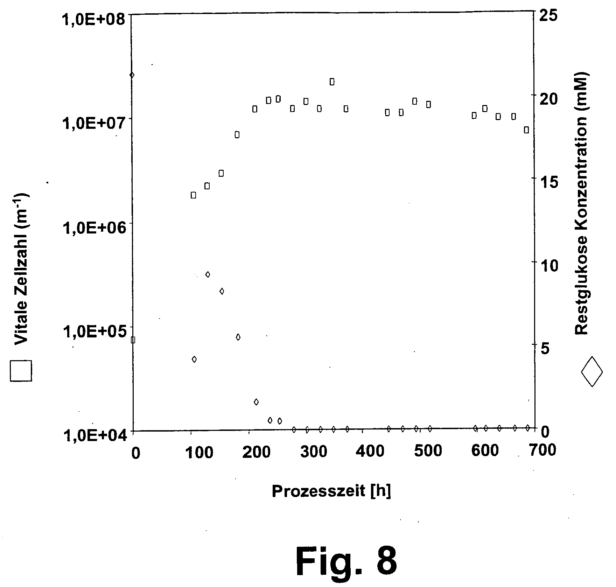

Figur 8 zeigt eine Graphik, in der auf der linken Seite die vitale Zellzahl [ml⁻¹] und auf der rechten Seite die Konzentration der Restglukose [mM] gegen die Prozesszeit [h] für die Produktion von MUC2-GFP-C-term in CHO MUC/IgG2a PH3744/25 aufgetragen ist. In ihr ist:
□ vitale Zellzahl und
0 Glukose.

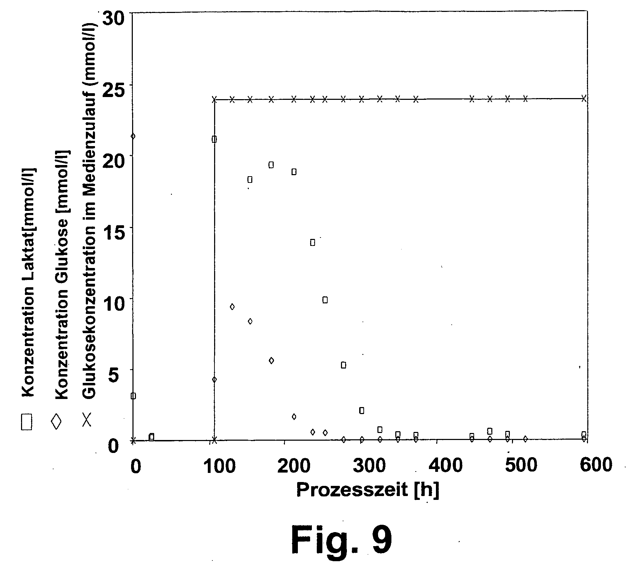

In Figur 9 ist die Glukose- und Laktatkonzentration sowie die Glukosekonzentration im Medienzulauf [mmol/l] gegen die Prozesszeit [h] aufgetragen. In ihr sind die Kurven mit
□ Laktatkonzentrationskurven und
0 Glukosekonzentrationskurven.
x 23,9 mmol/l Konzentration an Glukose im Medienzulauf (Durchflußrate von D = 0,035 h⁻¹).

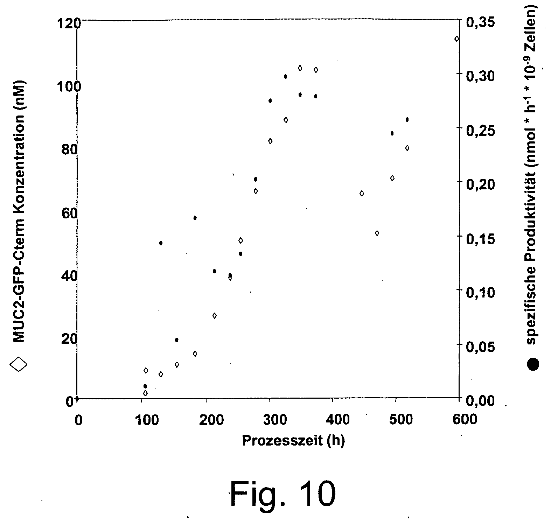

In Figur 10 ist die Konzentration von MUC2-GFP-C-term [nM] auf der linken Seite sowie qMUC2-GFP-C-term [nmol/(h*E9 Zellen)] auf der rechten Seite der Graphik gegen die Zeit [h] aufgetragen. In ihr sind:
• q spezifische Produktivität an MUC2-GFP-C-term (nmol/hE9 Zellen) und
◊ Konzentration an MUC2-GFP-C-term (nM).

Figur 1 zeigt eine Verfahrensweise beispielhaft betreffend die Glukosezufütterung. In kontinuierlicher Perfusionskultur wird eine konstante Menge Glukose zugefüttert. Im gezeigten Beispiel wird dies durch eine konstante Mediendurchflußrate erreicht, wobei die Glukosekonzentration im Medienzulauf konstant ist. Die Mediendurchflußrate wird nicht mit zunehmender Lebendzelldichte erhöht. Der Prozess wurde als Batch begonnen, bevor die kontinuierliche Verfahrensweise begann.

Figur 2 zeigt, daß bei dieser Verfahrensweise der DGL im Verlaufe des Prozesses sinkt, und schließlich einen Wert unter 0,4 erreicht. Während dies geschieht, steigt die spezifische Produktivität an und erreicht schließlich einen Wert der um das 4-fache höher ist, als der vor Unterschreiten des DGL-Wertes von 0,4.

Aus Figur 3 wird ersichtlich, dass bei dem erfindungsgemäßen Verfahren die Lebendzelldichte gegen einen Maximalwert läuft, der dann gehalten werden kann, während die Restglukosekonzentration im Verlauf gegen null geht. Dies tritt ein, obwohl Glukose zugeführt wird. Während des Absinkens der Restglukosekonzentration, beginnt die spezifische Glukoseaufnahmerate der Organismen zu sinken. Während dessen kann die Lebendzellzahl noch ansteigen. Parallel zum Rückgang der spezifischen Glukoseaufnahmerate sinkt auch die spezifische Laktatbildungsrate, was zunächst zu einem verlangsamten Anstieg, dann zu einem Abfall der Laktatkonzentration im Kulturgefäß führt. Schließlich läuft die Laktatkonzentration im Kulturgefäß gegen null, wie aus Figur 4 zu entnehmen ist. Es liegt also eine deutliche Umstellung des Zellmetabolismus vor. Wie Figur 5 zu entnehmen ist, findet verbunden mit der Umstellung des Zellmetabolismus ein Anstieg der spezifischen Produktivität auf etwa das 4-fache gegenüber dem Zeitpunkt vor der Umstellung des Zellmetabolismus statt. Der Anstieg der spezifischen Produktivität bei mindestens gleichbleibenden, oder sogar noch ansteigenden Zelldichten während der beschriebenen Phase führt schließlich zu einem markanten Anstieg des Produkttiters im Kulturüberstand, wie Figur 5 zu entnehmen ist, und damit zu einer erhöhten Raum-/Zeit-Ausbeute.

Tabelle 1 zeigt Daten zur Fermentation von MUC1-IgG2a.

Analog zu den Figuren 1 bis 5 beschreiben Figuren 6 bis 10 die Ergebnisse der Anwendung des erfindungsgemäßen Verfahrens mit CHO MUC2-GFP-C-term Zellen.

Tabelle 2 zeigt Daten zur Fermentation von MUC2-GFP-C-term. Produktionstechnisch kann das erfindungsgemäße Verfahren außer nach dem eben beschriebenen Perfusionsverfahren auch als Fed-Batch (Zufütterungsverfahren) betrieben werden.

In einem Fed-Batch-Betrieb wird die Produktionskultur einmal oder wiederkehrend, bzw. chargenweise oder kontinuierlich mit glukosehaltigem Medium oder einer separaten Glukoselösung in einer Art und Weise versorgt, daß der DGL vorzugsweise den Wert von 0,5, besonders bevorzugt 0,4 und noch besser 0,3 unterschreitet. Möglich ist hier auch ein repetitiver Fed-Batch.

Sowohl in der perfusiven Verfahrensweise als auch im Fed-Batch wird der Prozeß als Batch begonnen.

**Tabelle 1:**

| Prozesszeit | CV | D | Glukose Feed | Glukose | Laktat | MUC1-IgG2a | qMUC1-IgG2a | DGL |
|---|---|---|---|---|---|---|---|---|
| h | 1/ml | 1/h | mmol/l | mmol/l | mmol/l | µg/ml | µg/(h*E9) | |
| 0 | 2,23E+05 | 0 | 0 | 22,07 | 2,5 | 2,62 | | |
| 16,63 | 2,83E+05 | 0 | 0 | 20,89 | 5,1 | 3,59 | 0,21 | 0,92 |
| 40,52 | 6,48E+05 | 0 | 0 | 16,75 | 10,84 | 5,77 | 0,14 | 0,99 |
| 68 | 1,78E+06 | 0 | 0 | 8,74 | 20,1 | 14,21 | 0,17 | 0,61 |
| 94 | 2,14E+06 | 0,035 | 23,89 | 8,08 | 19,48 | 15,49 | 0,30 | 1,00 |
| 120 | 3,70E+06 | 0,035 | 23,89 | 5,84 | 22,35 | 18,02 | 0,22 | 0,72 |
| 136,5 | 4,68E+06 | 0,035 | 23,89 | 4,30 | 22,02 | 19,95 | 0,17 | 0,62 |
| 163,5 | 7,02E+06 | 0,035 | 23,89 | 3,17 | 22,66 | 22,67 | 0,14 | 0,40 |
| 187,5 | 6,96E+06 | 0,035 | 23,89 | 1,79 | 20,77 | 22,44 | 0,11 | 0,44 |
| 215,5 | 8,85E+06 | 0,035 | 23,89 | 1,04 | 17,46 | 28,24 | 0,13 | 0,35 |
| 264,75 | 1,30E+07 | 0,035 | 23,89 | - | 8,45 | 67,03 | 0,22 | 0,24 |
| 287 | 1,54E+07 | 0,035 | 23,89 | - | 5,25 | 89,42 | 0,22 | 0,20 |
| 310 | 1,64E+07 | 0,035 | 23,89 | - | 2,77 | 113,28 | 0,25 | 0,19 |
| 331 | 2,27E+07 | 0,035 | 23,89 | - | 1,24 | 133,80 | 0,24 | 0,14 |
| 352,4 | 1,45E+07 | 0,035 | 23,89 | - | 0,82 | 152,87 | 0,29 | 0,21 |
| 376,3 | 1,42E+07 | 0,035 | 23,89 | - | 0,53 | 182,52 | 0,45 | 0,22 |
| 404,4 | 1,58E+07 | 0,035 | 23,89 | - | 0,44 | 218,51 | 0,51 | 0,20 |
| 428 | 1,78E+07 | 0,035 | 23,89 | - | 0,58 | 241,75 | 0,50 | 0,17 |
| 448,4 | 2,08E+07 | 0,035 | 23,89 | - | 0,55 | 305,39 | 0,55 | 0,15 |
| 473,63 | 1,35E+07 | 0,035 | 23,89 | - | 0,55 | 290,52 | 0,60 | 0,23 |
| 496,8 | 9,30E+06 | 0,035 | 23,89 | - | 0,51 | 274,94 | 0,85 | 0,33 |
| 521,82 | 1,53E+07 | 0,035 | 23,89 | - | 0,56 | 301,12 | 0,87 | 0,20 |

| | | | | | | | | |
|---|---|---|---|---|---|---|---|---|
| Daten zur Fermentation von MUC1-IgG2a | | | | | | | | |

**Tabelle 2:**

| Prozeßzeit | Vitale Zellzahl | D | Glukose Feed | Glukose | Laktat | MUC2-GFP-C-term | qProdukt | DGL |
|---|---|---|---|---|---|---|---|---|
| h | 1/ml | 1/h | mmol/l | mmol/l | mmol/l | nM | nmol/(h*E9) | |
| 0,5 | 7,50E+04 | 0 | 0 | 21,37 | 3,12 | 0,00 | | |
| 106 | 1,80E+06 | 0 | 0 | 4,25 | 21,1 | 1,66 | 0,01 | 0,44 |
| 106,01 | | 0,035 | 23,89 | | | 8,92 | | |
| 130 | 2,20E+06 | 0,035 | 23,89 | 9,36 | | 7,71 | 0,14 | 0,66 |
| 154 | 2,90E+06 | 0,035 | 23,89 | 8,32 | 18,23 | 10,72 | 0,05 | 1,00 |
| 182,38 | 6,83E+06 | 0,035 | 23,89 | 5,58 | 19,28 | 14,08 | 0,17 | 0,53 |
| 212,9 | 1,19E+07 | 0,035 | 23,89 | 1,65 | 18,78 | 26,15 | 0,12 | 0,33 |
| 237,2 | 1,44E+07 | 0,035 | 23,89 | 0,54 | 13,84 | 38,37 | 0,11 | 0,26 |
| 254 | 1,48E+07 | 0,035 | 23,89 | 0,52 | 9,81 | 50,08 | 0,13 | 0,24 |
| 278 | 1,20E+07 | 0,035 | 23,89 | - | 5,19 | 65,63 | 0,20 | 0,35 |
| 302 | 1,40E+07 | 0,035 | 23,89 | - | 2,05 | 81,53 | 0,27 | 0,29 |
| 326 | 1,20E+07 | 0,035 | 23,89 | - | 0,7 | 88,03 | 0,30 | 0,34 |
| 349,9 | 2,16E+07 | 0,035 | 23,89 | - | 0,33 | 104,60 | 0,28 | 0,19 |
| 374 | 1,20E+07 | 0,035 | 23,89 | - | 0,26 | 104,03 | 0,28 | 0,34 |
| | | 0,035 | 23,89 | - | | 84,47 | | |
| | | 0,035 | 23,89 | - | | 75,16 | | |
| 446 | 1,10E+07 | 0,035 | 23,89 | - | 0,19 | 64,81 | | 0,37 |
| 470 | 1,10E+07 | 0,035 | 23,89 | - | 0,53 | 52,36 | | 0,37 |
| 494 | 1,40E+07 | 0,035 | 23,89 | - | 0,32 | 69,63 | 0,24 | 0,29 |
| 518 | 1,30E+07 | 0,035 | 23,89 | - | | 79,34 | 0,26 | 0,32 |
| | | 0,035 | 23,89 | - | | 93,94 | | |
| | | 0,035 | 23,89 | - | 0,35 | 104,57 | | |
| 595,8 | 1,01E+07 | 0,035 | 23,89 | - | 0,25 | 113,89 | | |

| | | | | | | | | |
|---|---|---|---|---|---|---|---|---|
| Daten zur Fermentation von MUC2-GFP-Cterm | | | | | | | | |

## Patentansprüche

1. Verfahren zur Kultivierung von CHO Zellen zur Produktion von Immunglobulinen oder Fragmenten von Immunglobulinen,
**dadurch gekennzeichnet,**
**daß** eine Substanzen produzierende CHO Zelle unter Glukoselimitierung DGL kultiviert wird,
wobei der Grad der Glukoselimitierung DGL = qGlc/qGlcₘₐₓ mit qGlc = beobachtete momentane spezifische Glukoseverbrauchsrate und qGlcₘₐₓ = maximal für diese CHO Zellen bekannten spezifischen Glukoseverbrauchsrate größer als der DGL ist, welcher zur ausschließlichen Erhaltung DGL_{Erhaltung} der CHO Zelle führt, und ≤ 0,5 ist,
wobei der DGL_{Erhaltung} = qGlc_{Erhaltung}/qGlcₘₐₓ ist, mit qGlc_{Erhaltung} = bei reinem Erhaltungsstoffwechsel beobachtete spezifische Glukoseverbrauchsrate und qGlcₘₐₓ = maximal für diese CHO Zellen bekannten spezifischen Glukoseverbrauchsrate, wobei die Menge zugefütterter Glukose nicht größer als 50 % dessen ist, was die ohne Glukoselimitierung maximal erwartete Zellzahl maximal verbrauchen kann,
wobei ein glukosehaltiges Medium eingesetzt wird, welches bezüglich anderer Nährstoffkomponenten nicht vor Eintreten der Glukoselimitierung limitierend ist, wobei das Verfahren als Batch gestartet und als kontinuierlicher Prozeß fortgeführt wird, wobei vor Beginn des kontinuierlichen Prozesses die Kultur als Batch betrieben wird.

2. Verfahren nach Anspruch 1, **dadurch gekennzeichnet, daß** der DGL ≤ 0,4 oder ≤ 0,3 ist.

3. Verfahren nach Anspruch 1 oder 2, **dadurch gekennzeichnet, daß** die Menge zugefütterter Glukose nicht größer als 35 % dessen ist, was die ohne Glukoselimitierung maximal erwartete Zellzahl maximal verbrauchen kann.

4. Verfahren nach einem der Ansprüche 1 bis 3, **dadurch gekennzeichnet, daß** die Glukose separat von anderen Substraten gefüttert wird.

5. Verfahren nach einem der Ansprüche 1 bis 4, **dadurch gekennzeichnet, daß** es in einem pH-Bereich von 6,7 - 7,7 durchgeführt wird.

6. Verfahren nach einem der Ansprüche 1 bis 5, **dadurch gekennzeichnet, daß** es in einem Temperaturbereich zwischen 35°C und 38,5°C durchgeführt wird.

7. Verfahren nach einem der Ansprüche 1 bis 6, **dadurch gekennzeichnet, daß** es in kontinuierlicher Verfahrensweise mit zumindest partieller Zellrückhaltung betrieben wird.

8. Verfahren nach einem der Ansprüche 1 bis 7, **dadurch gekennzeichnet, daß** es mit nicht wachstumsgekoppelt produzierenden Zellen durchgeführt wird.

9. Verfahren zur Kultivierung von CHO Zellen zur Produktion von Immunglobulinen oder Fragmenten von Immunglobulinen,
**dadurch gekennzeichnet,**
**daß** eine Substanzen produzierende CHO Zelle unter Glukoselimitierung DGL kultiviert wird,
wobei der Grad der Glukoselimitierung DGL = qGlc/qGlcₘₐₓ mit qGlc = beobachtete momentane spezifische Glukoseverbrauchsrate und qGlcₘₐₓ = maximal für diese CHO Zellen bekannten spezifischen Glukoseverbrauchsrate größer als der DGL ist, welcher zur ausschließlichen Erhaltung DGL_{Erhaltung} der CHO Zelle führt, und ≤ 0,5 ist,
wobei der DGL_{Erhaltung} = qGlc_{Erhaltung}/qGlcₘₐₓ ist, mit qGlc_{Erhaltung} = bei reinem Erhaltungsstoffwechsel beobachtete spezifische Glukoseverbrauchsrate und qGlcₘₐₓ = maximal für diese CHO Zellen bekannten spezifischen Glukoseverbrauchsrate, wobei die Menge zugefütterter Glukose nicht größer als 50 % dessen ist, was die ohne Glukoselimitierung maximal erwartete Zellzahl maximal verbrauchen kann,
wobei ein glukosehaltiges Medium eingesetzt wird, welches bezüglich anderer Nährstoffkomponenten nicht vor Eintreten der Glukoselimitierung limitierend ist, wobei das Verfahren aus einer Batch und einer Fed-Batch Verfahrensweise besteht, wobei das Verfahren als Batch gestartet und als Fed-Batch Prozeß fortgeführt wird.

10. Verfahren nach Anspruch 9, **dadurch gekennzeichnet, daß** der DGL ≤ 0,4 oder ≤ 0,3 ist.

11. Verfahren nach Anspruch 9 oder 10, **dadurch gekennzeichnet, daß** die Menge zugefütterter Glukose nicht größer als 35 % dessen ist, was die ohne Glukoselimitierung maximal erwartete Zellzahl maximal verbrauchen kann.

12. Verfahren nach einem der Ansprüche 9 bis 11, **dadurch gekennzeichnet, daß** die Glukose separat von anderen Substraten gefüttert wird.

13. Verfahren nach einem der Ansprüche 9 bis 12, **dadurch gekennzeichnet, daß** es in einem pH-Bereich von 6,7 - 7,7 durchgeführt wird.

14. Verfahren nach einem der Ansprüche 9 bis 13, **dadurch gekennzeichnet, daß** es in einem Temperaturbereich zwischen 35°C und 38,5°C durchgeführt wird.

15. Verfahren nach einem der Ansprüche 9 bis 14, **dadurch gekennzeichnet, daß** es mit nicht wachstumsgekoppelt produzierenden Zellen durchgeführt wird.

## Claims

1. Method of culturing CHO cells for the production of immunoglobulins or fragments of immunoglobulins,
**characterized in that**
a CHO cell producing substances is cultured under glucose limitation (DGL) conditions, wherein the degree of glucose limitation DGL = qGlc/qGlcₘₐₓ, where qGlc = observed actual specific rate of glucose consumption, and qGlcₘₐₓ = known maximum specific rate of glucose consumption for said CHO cells, is greater than the DGL resulting exclusively in CHO cell maintenance, DGL_{maintenance}, and is ≤ 0.5,
with DGL_{maintenance} = qGlc_{maintenance}/qGlcₘₐₓ, where qGlC_{maintenance} = observed specific rate of glucose consumption for maintenance metabolism only and qGlcₘₐₓ = known maximum specific rate of glucose consumption for said CHO cells,
wherein the amount of glucose fed in is no more than 50% of what the maximum number of cells expected without glucose limitation is at most able to consume,
wherein a glucose-containing medium is employed that is not limiting with regard to other nutrient components until glucose limitation sets in,
wherein the method begins by way of a batch process and continues by way of a continuous process, with the batch culture being operated before the continuous process commences.

2. Method according to Claim 1, **characterized in that** DGL ≤ 0.4 or ≤ 0.3.

3. Method according to Claim 1 or 2, **characterized in that** the amount of glucose fed in is no more than 35% of what the maximum number of cells expected without glucose limitation is at most able to consume.

4. Method according to any of Claims 1 to 3, **characterized in that** the glucose is fed separately from other substrates.

5. Method according to any of Claims 1 to 4, **characterized in that** it is carried out within a pH range of 6.7 - 7.7.

6. Method according to any of Claims 1 to 5, **characterized in that** it is carried out within a temperature range of between 35°C and 38.5°C.

7. Method according to any of Claims 1 to 6, **characterized in that** it is carried out by way of a continuous procedure with at least partial cell retention.

8. Method according to any of Claims 1 to 7, **characterized in that** it is carried out using cells producing in a non-growth-coupled manner.

9. Method of culturing CHO cells for the production of immunoglobulins or fragments of immunoglobulins,
**characterized in that**
a CHO cell producing substances is cultured under glucose limitation (DGL) conditions, wherein the degree of glucose limitation DGL = qGlc/qGlcₘₐₓ, where qGlc = observed actual specific rate of glucose consumption, and qGlcₘₐₓ = known maximum specific rate of glucose consumption for said CHO cells, is greater than the DGL resulting exclusively in CHO cell maintenance, DGL_{maintenance}, and is ≤ 0.5,
with DGL_{maintenance} = qGlC_{maintenance}/qGlCₘₐₓ, where qGlc_{maintenance} = observed specific rate of glucose consumption for maintenance metabolism only and qGlcₘₐₓ = known maximum specific rate of glucose consumption for said CHO cells,
wherein the amount of glucose fed in is no more than 50% of what the maximum number of cells expected without glucose limitation is at most able to consume,
wherein a glucose-containing medium is employed that is not limiting with regard to other nutrient components until glucose limitation sets in,
wherein the method consists of a batch and a fed-batch procedure,
wherein the method begins by way of a batch process and continues by way of a fed-batch process.

10. Method according to Claim 9, **characterized in that** DGL ≤ 0.4 or ≤ 0.3.

11. Method according to Claim 9 or 10, **characterized in that** the amount of glucose fed in is no more than 35% of what the maximum number of cells expected without glucose limitation is at most able to consume.

12. Method according to any of Claims 9 to 11, **characterized in that** the glucose is fed separately from other substrates.

13. Method according to any of Claims 9 to 12, **characterized in that** it is carried out within a pH range of 6.7 - 7.7.

14. Method according to any of Claims 9 to 13, **characterized in that** it is carried out within a temperature range of between 35°C and 38.5°C.

15. Method according to any of Claims 9 to 14, **characterized in that** it is carried out using cells producing in a non-growth-coupled manner.

## Revendications

1. Procédé de culture de cellules CHO pour la production d'immunoglobulines ou de fragments d'immunoglobulines,
**caractérisé en ce qu'**une cellule CHO produisant des substances est cultivée sous limitation du glucose DGL,
dans lequel le degré de limitation du glucose DGL = qGlc/qGlcₘₐₓ, avec qGlc = taux de consommation du glucose spécifique momentané observé et qGlcₘₐₓ = taux de consommation du glucose spécifique maximal connu pour ces cellules CHO, est supérieur au DGL conduisant à l'obtention exclusive DGL_{obtention} de la cellule CHO et est ≤ 0, 5,
dans lequel DGL_{obtention} = qGlc_{obtention}/qGlcₘₐₓ, avec qGlc_{obtention} = taux de consommation du glucose spécifique observé en cas de métabolisme d'obtention pur et qGlcₘₐₓ = taux de consommation du glucose spécifique maximal connu pour ces cellules CHO, la quantité de glucose apporté n'excédant pas 50 % de ce que peut consommer au maximum le nombre maximal prévu de cellules sans limitation du glucose,
dans lequel on met en oeuvre un milieu contenant du glucose qui n'est pas limitant vis-à-vis des autres composants nutritifs avant l'instauration de la limitation du glucose,
ledit procédé étant lancé en mode batch et poursuivi sous forme continue, la culture étant effectuée sous forme de lots avant le début du procédé continu.

2. Procédé selon la revendication 1, **caractérisé en ce que** le DGL est ≤ 0,4 ou ≤ 0,3.

3. Procédé selon la revendication 1 ou 2, **caractérisé en ce que** la quantité de glucose apporté n'excède pas 35 % de ce que peut consommer au maximum le nombre maximal prévu de cellules sans limitation du glucose.

4. Procédé selon l'une des revendications 1 à 3, **caractérisé en ce que** le glucose est apporté séparément des autres substrats.

5. Procédé selon l'une des revendications 1 à 4, **caractérisé en ce qu'**il est réalisé dans une plage de pH de 6,7 à 7,7.

6. Procédé selon l'une des revendications 1 à 5, **caractérisé en ce qu'**il est réalisé dans une plage de températures situées entre 35°C et 38,5°C.

7. Procédé selon l'une des revendications 1 à 6, **caractérisé en ce qu'**il est réalisé en mode continu avec une rétention au moins partielle des cellules.

8. Procédé selon l'une des revendications 1 à 7, **caractérisé en ce qu'**il est réalisé avec des cellules de production non couplée à la croissance.

9. Procédé de culture de cellules CHO pour la production d'immunoglobulines ou de fragments d'immunoglobulines,
**caractérisé en ce qu'**une cellule CHO produisant des substances est cultivée sous limitation du glucose DGL,
dans lequel le degré de limitation du glucose DGL = qGlc/qGlcₘₐₓ, avec qGlc = taux de consommation du glucose spécifique momentané observé et qGlcₘₐₓ = taux de consommation du glucose spécifique maximal connu pour ces cellules CHO, est supérieur au DGL conduisant à l'obtention exclusive DGL_{obtention} de la cellule CHO et est ≤ 0, 5,
dans lequel DGL_{obtention} = qGlc_{obtention}/qGlcₘₐₓ, avec qGlc_{obtention} = taux de consommation du glucose spécifique observé en cas de métabolisme d'obtention pur et qGlcₘₐₓ = taux de consommation du glucose spécifique maximal connu pour ces cellules CHO, la quantité de glucose apporté n'excédant pas 50 % de ce que peut consommer au maximum le nombre maximal prévu de cellules sans limitation du glucose,
dans lequel on met en oeuvre un milieu contenant du glucose qui n'est pas limitant vis-à-vis des autres composants nutritifs avant l'instauration de la limitation du glucose, ledit procédé étant constitué d'un mode batch et d'un mode fed-batch, ledit procédé étant lancé en mode batch et poursuivi sous forme fed-batch.

10. Procédé selon la revendication 9, **caractérisé en ce que** le DGL est ≤ 0,4 ou ≤ 0,3.

11. Procédé selon la revendication 9 ou 10, **caractérisé en ce que** la quantité de glucose apporté n'excède pas 35 % de ce que peut consommer au maximum le nombre maximal prévu de cellules sans limitation du glucose.

12. Procédé selon l'une des revendications 9 à 11, **caractérisé en ce que** le glucose est apporté séparément des autres substrats.

13. Procédé selon l'une des revendications 9 à 12, **caractérisé en ce qu'**il est réalisé dans une plage de pH de 6,7 à 7,7.

14. Procédé selon l'une des revendications 9 à 13, **caractérisé en ce qu'**il est réalisé dans une plage de températures situées entre 35°C et 38,5°C.

15. Procédé selon l'une des revendications 9 à 14, **caractérisé en ce qu'**il est réalisé avec des cellules de production non couplée à la croissance.
